# EUROPEAN PATENT APPLICATION

(11) **EP 4 122 330 A1**
(43) Date of publication of application: **25.01.2023**
(21) Application number: 21187183.5
(22) Date of filing: 22.07.2021
(51) Int. Cl.: A23L 17/60, A61K 36/02, A61K 36/05

(54) **METHODS OF GROWING MICROALGAE & PRODUCTS THEREOF**

(71) Applicant: Alver World SA, 1566 Saint-Aubin (CH)
(72) Inventor: URAN, Mine, 1803 Chardonne (CH); CAPORGNO, Martín, 8004 Zürich (CH); ZINN, Manfred, 1967 Bramois (CH); TREMBLAY, Priscilla, 1950 SION (CH); POTT, Julien, 1950 SION (CH)
(74) Representative: reuteler & cie SA

(57) **Abstract**

The present invention relates to a method of preparation of comprising microalgae, in particular *Chlorella* and products resulting thereof.

## Description

### Field of the invention

The present invention relates to a method for growing microalgae, in particular *Chlorella* genus, more preferably *Chlorella vulgaris* and the resulting microalgae products.

### Background

Society is becoming increasingly conscious of sustainable nutrition as the world's population keeps rising (FAO, 2017: The future of food and agriculture. Trends and challenges. Rome: Food and Agriculture Organization of the United States)*.* The unpredictability of the future of food production with respect to water and land usage, in addition to the changing in consumer preferences, brings the need for additional food sources having a high nutritional quality (van Huis et al., 2014, Edible insects - Future prospects for food and feed security. Rome: FAO*).* As the negative impact of animal rearing on the environment such as shortage of water, stripping the land of nutrients, and increase of pollution (antibiotics for livestock) became fully aware in our society, a more environmentally friendly lifestyle has been targeted by vegetarian and vegan population. Thus, there is a growing need for especially protein-rich foods since vegans do not have access to animal proteins (e.g., eggs) (*van Huis et al., 2014, supra; FAO, 2017, supra;* Henchion et al 2017; Foods, 6 (7). DOI: 10.3390/foods6070053)*.* Current protein sources are not fulfilling the growing demand for low environmental impact and high protein sources (*Henchion et al, 2017, supra*)*.*

For decades, microalgae have been recognized as superfood for being both healthy and sustainable (Vrenna et al., 2021, Sustainability 13 (5), p. 2848. DOI: 10.3390/su13052848; Kusmayadi et al., 2021, Chemosphere 271, p. 129800. DOI: 10.1016/j.chemosphere.2021.129800*;* Koyande et al. 2019, Food Science and Human Wellness, 8 (1), 16-24. DOI: 10.1016/j.fshw.2019.03.001*).* Microalgae-based proteins require less land and water compared to animal-based proteins and for their cultivation, non-arable land is used (Caporgno et al., 2018 Frontiers in nutrition, 5, p. 58. DOI: 10.3389/fnut.2018.00058)*.* Today, microalgae are considered as promising sustainable alternative protein sources for the future. Microalgae are rich sources of protein (more than 50%) and have well-balanced amino acid profiles regarding the recommendations of the WHO/FAO/UNU (Vieira et al., 2020, New and Future Developments in Microbial Biotechnology and Bioengineering, 54: Elsevier, 19-30*;* FAO/WHO/UNU Expert Consultation on Energy and Protein Requirements. AMINO ACID SCORING PATTERNS (1981*). With assistance of A. Harper. Rome. Available online at http:*//*www.fao.org*/*3*/*M3013EIM3013E00.htm, checked on 28*/*06*/*2021).*

Several microalgae species are widely used as nutritional supplements in tablet, capsule, or powdered form due to their taste, color, smell, and cost (Lafarga, 2019, Algal Research, 41, 101566. DOI: 10.1016/j.algal.2019.101566). The bacterium *Spirulina* and the microalgae *Chlorella* are two of the commercially important species on the market today (Nilesh Hemantkumar et al., 2020 Milada Vítová (Ed.): Microalgae - From Physiology to Application: IntechOpen)*,* especially due to their high protein contents (Andrade, 2018, MOJFPT 6 (1). DOI: 10.15406/mojfpt.2018.06.00144)*.* Microalgae contain also other healthy compounds besides proteins that can be used for several applications (Barkia et al., 2019 Marine drugs, 17 (5). DOI: 10.3390/md17050304*;* Milledge, 2011, Rev. Environ. Sci. Biotechnol., 10.1007/s11157-010-9214-7). Even if large-scale cultivation of *Chlorella vulgaris* is a consequence of the interest on the protein fraction accumulated and the content of essential amino acids, other beneficial nutrients such as vitamins (B-complex and ascorbic acid), minerals (potassium, sodium, magnesium, iron, and calcium), and pigments (β-carotene and chlorophyll) can be exploited using these microalgae (Becker, 2007 Biotechnology advances, 25 (2), 207-210. DOI: 10.1016/j.biotechadv.2006.11.002*;* Rodriguez-Garcia et al., 2008, Food Chem., 108, 1023-1026*).*

On the other hand, as obtained from a microalga, *Chlorella* products have a green color, a bitter taste and a strong smell because of their chlorophyll content. Their application in the food industry is limited, especially in those products where the color of the microalgae cannot be masked with other ingredients, like in dairy products. In some food products, microalgae are also used as natural food colorants thanks to their pigments (*Becker, 2007, supra;* Caporgno et al., 2018, Frontiers in nutrition 5, 58. DOI: 10. 3389/fnut.2018. 00058)*.*

*C. vulgaris* is growing fast and therefore cultivation techniques can be tailored to optimize protein content and biomass yield (Fransiscus et al., 2017, AIP conference Proceedings, 1840, 030005*).* The reason is that different growth conditions cause C. *vulgaris* to modify the yield of biomass concerning protein, lipid, carbohydrate, and/or pigment content (Ibrahim et al., 2020, AJVS 65, (1), 16. DOI: 10.5455/ajvs.94847*).* Depending on the strain, C. *vulgaris* can be grown autotrophically, heterotrophically or mixotrophically (Iwamoto, 2003 Amos Richmond (Ed.): Handbook of Microalgal Culture. Oxford, UK: Blackwell Publishing Ltd, 253-263*,* Fatemeh et al., 2016, Journal of Applied Sciences and Environmental Management, 20 (1), 133. DOI: 10.4314/jasem.v20i1.16*).* Autotrophically, C. *vulgaris* are usually grown in open pond systems (photosynthetically) for large-scale biomass production (autotrophic conditions) as open pond systems are cheap in maintenance (*Ibrahim et a*/*., 2020, supra.*). Heterotrophic (dark) growth conditions are found to reduce the chlorophyll content (Khan et al., 2016, Heterotrophic Growth of Micro Algae. In J. Liu, Z. Sun, H. Gerken (Eds.): Recent Advances in Microalgal Biotechnology) which can be a promising solution regarding the customer acceptance (Boeing et al., 2015, 12th European Nutrition Conference (FENS), Berlin, Germany, October 20-23, 2015: Abstracts. In Annals of nutrition & metabolism 67 Suppl 1, 1-601. DOI: 10.1159/000440895)*.* The heterotrophic growth technique is a process in which microalgae are grown on an organic carbon source, such as glucose, glutamate, and glycerol as well as on additional nutrients (Brennan et al., 2010 Renewable and Sustainable Energy Reviews, 14 (2), 557-577. DOI: 10.1016/.rser.2009.10.009*;* Nikodinovic-Runic et al., 2013 Advances in applied microbiology, 84, 139-200. DOI: 10.1016/B978-0-12-407673-0.00004-7). Heterotrophic growth techniques instead of open pond systems allow to achieve a faster growth, higher dry biomass productivity, high yield of different components like proteins or lipids (*Ibrahim et al., 2020, supra.;* Hu et a/., 2018, Biotechnology Advances 36, 54-67). Chlorophyll content is also reduced in heterotrophic conditions due to the absence of light (*Khan et a*/*., 2016, supra*)*.* Microalgae cells first grown heterotrophically significantly accumulate chlorophyll when later on exposed to light *(*Ogbonna et al., 1997, Journal of Applied Phycology, 9, 359-366, doi: 10.10231A:1007981930676*).*

In terms of costs, compared to autotrophic production, heterotrophic production can use cheap nutrients, requires lower costs of instruments (mainly due to high costs of material that allows thorough light penetration in autotrophic systems) and financial investment for energy use and maintenance, and the easy operation and maintenance of the system also has a beneficial impact on the reproducibility of production and the biomass costs (Jareonsin et al., 2021, Front. Bioeng. Biotechnol., 9:628597. doi: 10.3389/fbioe.2021.628597). However, the cost of the organic carbon source still represents one of the major costs for heterotrophic cultivation (for example when glucose or acetate is used); therefore, finding cheap sources can have a strong impact on the biomass costs (*Hu et al., 2018, supra*).

Molasses, as a residue from sugar processing, is a cheap, tasty source of fermentable carbohydrates and is considered as a valuable by-product that can be used as carbon source in different fermentation processes (*Nikodinovic-Runic, et al., 2013, supra*)*.* Molasses can be also used for microalgae cultivation. However, dissimilar results can be found in the literature that could be attributed to the different strains but also the cultivation conditions. Some *Chlorella* species like *Chlorella protothecoides* have shown the ability to grow in hydrolyzed molasses as the microalgae was not able to metabolize sucrose, and also other hydrolyzed carbon sources (Yan, et al., 2011, Bioresour. Technol., 102, 6487-6493). Regarding C. *vulgaris,* different carbon sources have been used for cultivation. For example, *Fransiscus and Purwanto* (*Fransiscus et al., 2017, supra*) evaluated glucose, fructose and sucrose at four different concentrations as single carbon sources for the growth of C. *vulgaris* and indicated that the three carbon sources are consumed by C. *vulgaris* and showed no significant difference in the growth pattern. Contrary to results reported by *Fransiscus and Purwanto (2017), Vidotti et al.* observed that cultivation with fructose resulted in no growth but rather in cell death, indicating that this strain was unable to consume fructose (Vidotti et al., 2014, Chemical Engineering Transactions, 38, 325-330 DOI: 10.3303/CET143805*).*

Consequently, there is a need for cost-effective innovative production techniques of C. *vulgaris* with lower chlorophyll content with suitable taste for food applications and adapted to large scale production.

### Summary of the Invention

An object of this invention is to provide a method for growing microalgae, preferably *Chlorella* genus, more preferably *Chlorella vulgaris.*

It is advantageous to provide a method which allows the production of microalgae under heterotrophic growth conditions with a high volumetric biomass productivity (e.g., high specific growth rate and high cell density).

It is advantageous to provide a method which allows the production of microalgae under heterotrophic growth conditions using organic carbon sources at low cost.

It is advantageous to provide a method which allows the production of microalgae under heterotrophic growth conditions which are environmentally friendly and consumes less water.

It is advantageous to provide a method which allows the production of microalgae suitable for human consumption as food ingredient and/or a food supplement and has a reduced chlorophyll content in the microalgal biomass and an increased fraction of carotenoids in the pigments.

It is advantageous to provide a method which allows the production of microalgae suitable for human consumption as food ingredient and/or a food supplement and has a sweeter taste due to the presence of fructose in the final product.

Objects of this invention have been achieved by providing a method according to claim 1.

Objects of this invention have been achieved by providing a microalgae material according to claim 13 or 14, a food or feed product according to claim 14.

Disclosed herein, according to a first aspect of the invention, is a method for production of microalgae under heterotrophic fermentation conditions, wherein said method comprises the step of stopping the fermentation process when the fructose content in the microalgae culture medium reaches the target level, said target level being at least 1 g/L.

Disclosed herein, according to a first aspect of the invention, is a method for production of microalgae, wherein said method comprises the following steps:
- Providing a microalgal inoculum to a microalgae culture medium;
- Supplying the microalgae culture medium with at least 1 g/L of fructose;
- Incubating the microalgae under heterotrophic fermentation conditions in the culture medium;
- Stopping the fermentation process when the fructose concentration of the microalgae culture medium is at least 1 g/L;
- Harvesting the microalgae material from the culture medium;
- Optionally washing the harvested microalgae material to obtain a concentrated microalgae material with a fructose content that is not less than about 0.2% w/w.

Disclosed herein, according to a further aspect of the invention, is a microalgae product suitable for human consumption, containing at least 0.2 % w/w fructose and high fraction of carotenoids in the pigments compared to the microalgae product produced in heterotrophic conditions without fructose.

It is advantageous to provide a microalgae product suitable for human consumption with reduced chlorophyll pigment compared to the microalgae product produced by known for heterotrophic conditions and/or in presence of light.

The invention is based on the unexpected finding that a method for production of microalgae comprising the supplementation in fructose to the microalgae culture medium allowing the presence of at least 0.2% w/w fructose in the final microalgae product, provides nutritional advantages such as a reduced chlorophyll content and a higher fraction of carotenoids in the pigments compared to a microalgal biomass produced in absence of fructose. The final microalgae product, suitable for human consumption, according to the invention also provides nutritional advantages such as a reduced bitterness.

It was also unexpectedly found that the presence of fructose in the culture medium increases the biomass yield compared to the culture when fructose was absent or when used temporarily only during the washing step.

Other features and advantages of the invention will be apparent from the claims, detailed description, and figures.

### Description of the figures

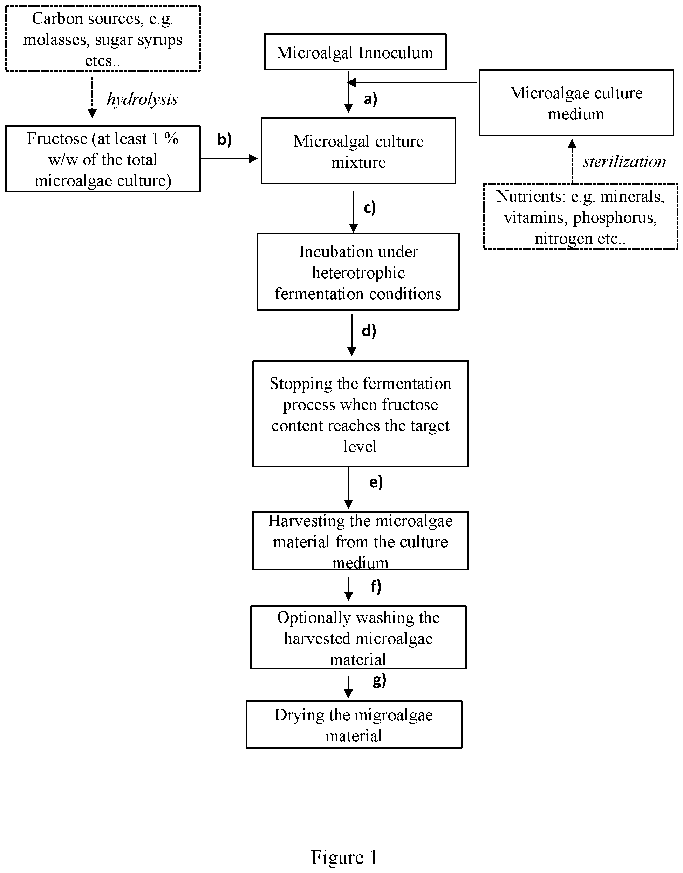

**Figure** 1 shows schematized steps of a method according to the invention.

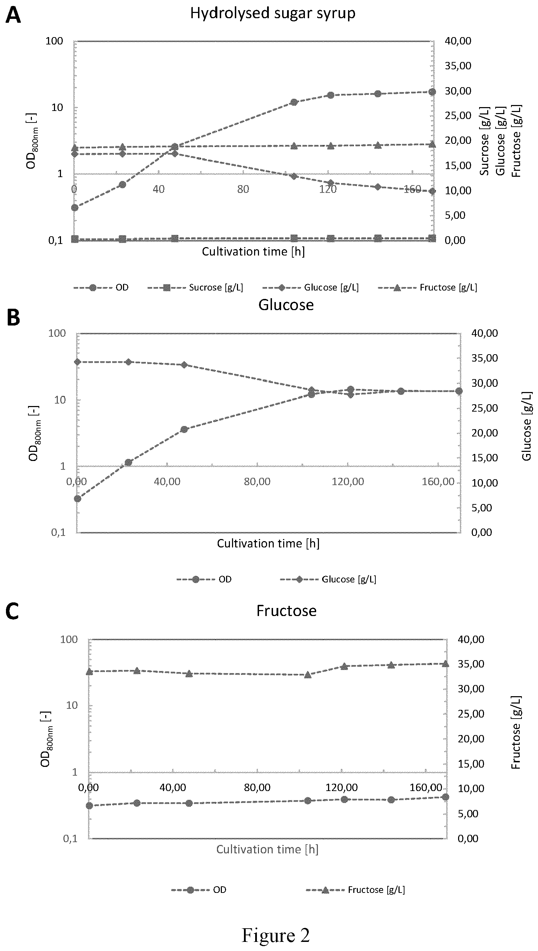

**Figure** 2 shows the C. *vulgaris* growth in a method according to the invention, e.g., in hydrolyzed sugar syrup containing fructose (A), compared to other conditions with a single source of carbon such as glucose (B) and fructose (C). Growth is monitored through the absorbance of the culture medium at 800 nm and the variation of the sugar content in the culture medium is measured by HPLC. OD₈₀₀ₙₘ: optical density at 800 nm.

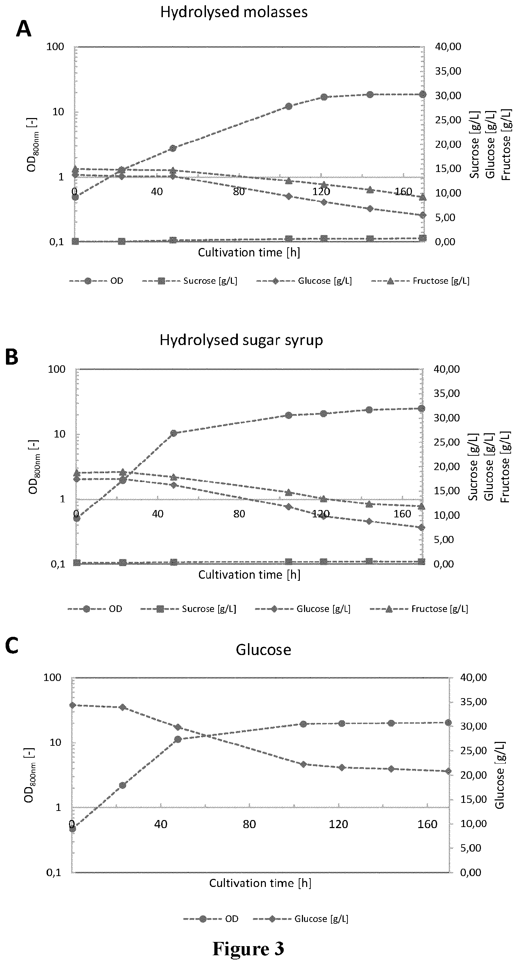

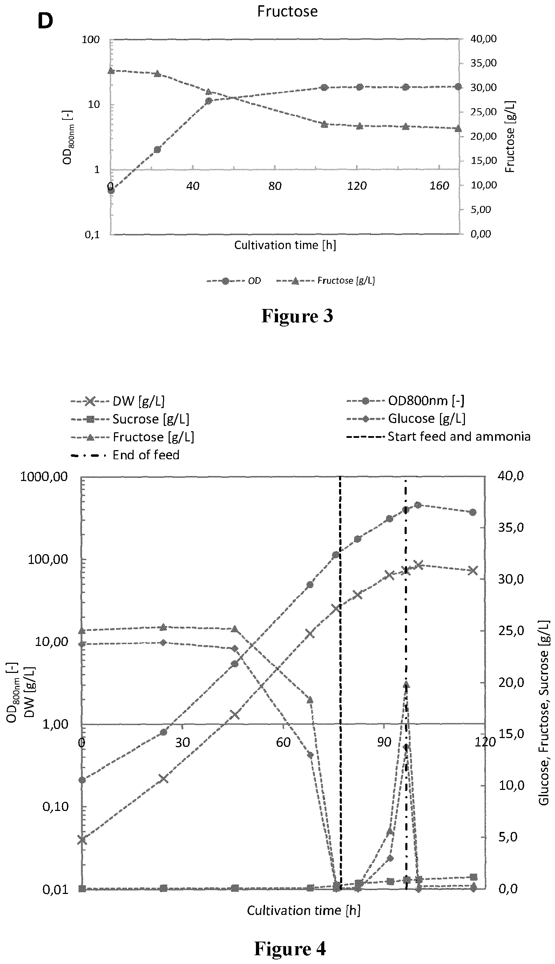

**Figure** 3 shows the growth of C. *protothecoides* growth in a method according to the invention using hydrolyzed molasses and hydrolyzed sugar syrup, both containing fructose (A and B), compared to other conditions with a single source of carbon such as glucose (C) and fructose (D). Growth is monitored through the absorbance at 800 nm and the variation of the sugar content in the culture medium is measured by HPLC.
**Figure** 4 shows the *C. protothecoides* growth during a fed-batch cultivation using an exponential feed of hydrolyzed sugar syrup. Growth is monitored through the absorbance at 800 nm and through the measurement of dry cell weight. The variation of the sugar content in the culture medium is measured by HPLC.

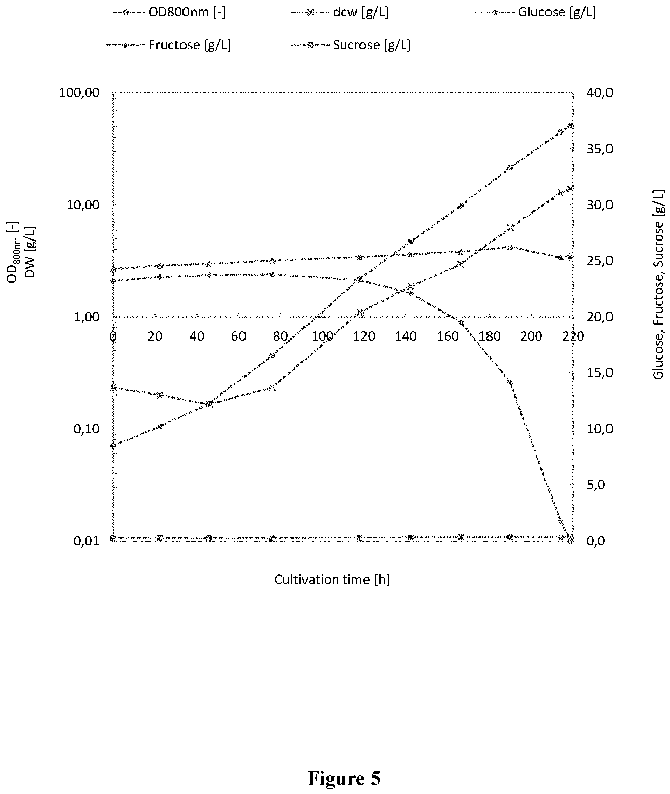

**Figure** 5 shows the C. *vulgaris* growth during a batch cultivation with hydrolyzed sugar syrup as a carbon source. Growth is monitored through the absorbance at 800 nm and through the measurement of dry cell weight. The variation of the sugar content in the culture medium is measured by HPLC.

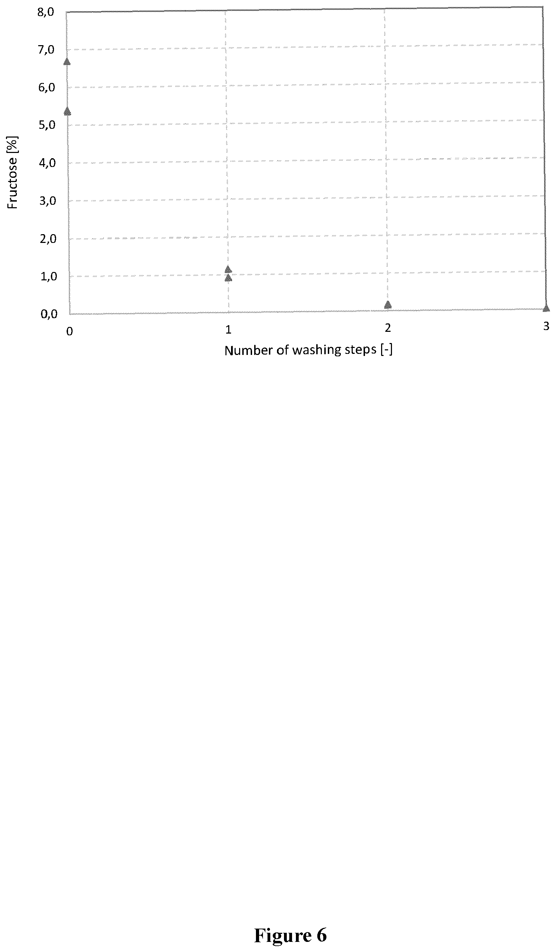

**Figure** 6 compares various washing conditions as described in Example 4.

### Detailed description

"Microalgae" refers to microscopic, eukaryotic single cells composed of a nucleus, one or more chloroplasts, mitochondria, Golgi bodies, endoplasmic reticulum and other organelles. Microalgae suitable in the context to the invention belong to the phylum *Chlorophyta,* preferably *Chlorella* genus, more preferably *Chlorella vulgaris.* Those include species such as *Chlorella protothecoides, Chlorella ellipsoidea, Chlorella minutissima, Chlorella Zofingiensis, Chlorella luteoviridis, Chlorella kessleri, Chlorella sorokiniana, Chlorella fusca var. vacuolata, Chlorella sp., Chlorella cf. minutissima* or *Chlorella emersonii.*

Other species of *Chlorella* can be selected from the group consisting of *Anitrata, Antarctica, aureoviridis, candida, capsulate, desiccate, ellipsoidea* (including strain CCAP 211/42), *emersonii, fusca* (including *var. vacuolata*)*, glucotropha, infusionum* (including *var. actophila* and *var. auxenophila*), *kessleri* (including any of UTEX strains 397,2229,398), *lobophora* (including strain SAG 37.88), *luteoviridis* (including strain SAG 2203 and *var. aureoviridis and lutescens*), *miniata, cf. minutissima, minutissima* (including UTEX strain 2341), *mutabilis, nocturna, ovalis, parva, photophila, pringsheimii, protothecoides* (including any of UTEX strains 1806, 411 , 264, 256, 255, 250, 249, 31, 29, 25 or CCAP 211/8D, or CCAP 211/17 and *var. acidicola*), *regularis* (including *var. minima,* and *umbricata*)*, reisiglii* (including strain CCP 11/8), *saccharophila* (including strain CCAP 211/31, CCAP 211/32 and *var. ellipsoidea*), *salina, simplex, sorokiniana* (including strain SAG 211.40B), *sp.* (including UTEX strain 2068 and CCAP 211/92), *sphaerica, stigmatophora, trebouxioides, vanniellii, vulgaris* (including strains CCAP 211/1 IK, CCAP 211/80 *and f. tertia* and *var. autotrophica, viridis, vulgaris, vulgaris f. tertia, vulgaris f. viridis*), *xanthella,* and *zofingiensis.*

The microalgal material can have a reduced green coloration compared to the microalgal material produced in a process different to the one described in the present invention, or be non-green such as yellow, or yellow-white or optionally white when the microalgae strain used was subjected to bioengineering processes such as random mutagenesis, aiming to generate mutant strains with altered pigment contents (Saini et al., 2020, Crit Rev Food Sci Nutr., 60(3):391-405. doi: 10.1080/10408398.2018.1533518. Epub 2019 Feb 1. PMID: 30706720*).*

The expression "microalgal inoculum" refers to the liquid culture containing microalgae meant to be introduced in a medium able to carry out its optimal multiplication for studying purposes or in order to produce bigger quantities.

The expression "biomass yield" refers to the ratio between the amount of dry biomass produced for a quantity of substrate consumed.

The term "fed batch" is a semi-continuous method during which nutrients are fed into the reactor during the cultivation.

The expression "exponential feeding" during cultivation refers to a feeding mode used for nutrients in the medium, so that exponentially growing organisms remain in a prolonged exponential phase with a defined growth rate.

The expression "Chlorophyll" refers to the principal pigment involved in photosynthesis. It allows for the photosynthetic organism to absorb the energy of the light in order to convert CO₂ into carbohydrates necessary for its growth. This pigment can be found in different forms: Chlorophyll a, b, c and d (and sometimes but rarely e). It is formed by a central magnesium atom surrounded by a porphyrin ring (nitrogen containing structure). The content of chlorophyll in microalgae can be determined by different methods, most of them involving the extraction of the pigments for example with organic solvents such as acetone, methanol, ethanol, etc., followed by a quantification using spectrophotometric analysis. Several methods can be found in the literature, such as the method described by Pruvost et al., Bioresource Technology, 102(1), 150-158. doi:10.016/j.biortech.2010.06.153*.*

The expression "fraction of carotenoids in the total pigments" refers to the ratio between the concentration of carotenoids and the total concentration of pigments, being carotenoids and chlorophylls.

The expression "heterotrophic fermentation conditions" refers to conditions for cell growth and propagation using an external carbon source under dark conditions. Several examples of microalgae growth under heterotrophic conditions, using different organic carbon sources, are summarized in the literature (Hu et al., 2018, Biotechnology Advances 36, 54-67).

A *"microalgae culture medium"* suitable for heterotrophic fermentation conditions comprises i) a carbon source and ii) nutrients for microalgal growth. According to the present invention, the carbon source is either fructose alone (100% w/w of the carbon source) or comprises a mixture with various carbon sources such as glucose, fructose and/or complex mixtures of sugars generated during hydrolysis of different carbon sources. The microalgae culture medium is supplied with fructose in order to obtain a fructose content in the culture medium is not less than 1% w/w at the end of the fermentation process. Fructose can be provided through the artificial addition of fructose or after hydrolysis of molasses or sugar syrups which are for example sugar-processing by-products.

Nutrients for microalgae growth suitable for heterotrophic fermentation conditions are known to the skilled person and can be found for example under https://utex.org/pages/algal-culture-media (University of Texas at Austin for its algal culture collection (UTEX)).

The microalgal biomass is itself a finished food ingredient and may be used in foodstuffs without further, or with only minimal, modification. Alternatively, after concentration, microalgal biomass can be processed to produce different food or feed products containing microalgae as an ingredient aiming to increase the nutritional value (pasta, drinks, meat substitutes, etc.) and/or as food supplements (in form of capsules, pills, etc). The microalgal biomass obtained according to a method of the invention can also be used for the extraction of high-value compounds, such as carotenoids.

Referring to the figures, in particular first to **Figure** 1, is provided an illustration of a method for production of microalgae comprising a step of stopping the fermentation process under heterotrophic fermentation conditions when the fructose content in the microalgae culture medium reaches the target level, said target level being at least 1 g/L but lower than a concentration of fructose that would inhibit the microalgae growth. The maximum concentration level that causes growth inhibition has to be determined for a specific strain used for cultivation. The illustrated method for the production of microalgae generally comprises the steps of: providing a microalgal inoculum to a microalgae culture medium wherein said medium is supplied with fructose, incubating the microalgae under heterotrophic fermentation conditions in the culture medium, stopping the fermentation process when the fructose content of the microalgae culture medium reaches the target level, said target level being at least 1 g/L of fructose, harvesting the microalgae material, and optionally washing the harvested microalgae material, optionally with a fructose containing washing solution, to obtained an isolated microalgae material with a fructose content that is not less than about 0.2% w/w.

More specifically, the steps of the embodiment illustrated in Figure 1 comprise:
- Providing a microalgal inoculum to a microalgae culture medium;
- Supplying the microalgae culture medium with at least 1 g/L of fructose;
- Incubating the microalgae under heterotrophic fermentation conditions in the culture medium;
- Stopping the fermentation process when the fructose concentration of the microalgae culture medium is at least 1 g/L;
- Harvesting the microalgae material from the culture medium;
- Optionally washing the harvested microalgae material to obtain a concentrated microalgae material with a fructose content that is not less than about 0.2% w/w.

According to a particular embodiment, an inoculum is provided of about 1% v/v.

According to a particular embodiment, the fructose can be supplied in the form of a carbon source which contains fructose and/or a carbon source that generates fructose when hydrolyzed.

According to a further particular embodiment, the fructose can be provided by a source of carbon which contains sucrose and/other saccharides and wherein said source of carbon is provided to the culture medium after hydrolysis such as for example described herein. According to another embodiment, the fructose can be provided through the direct addition of fructose in the culture medium.

According to another embodiment the carbon source is selected from the group consisting of fructose, sucrose, sugar syrup, molasses, sugar cane, sugar cane bagasse, sugar beet, beetroot juice, sugar maple sap, etc. that can be use in food applications.

According to another embodiment, the culture medium contains different nutrients required for microalgae growth, selected based on the microalgae strain used. In particular, nitrogen and phosphorus are considered as major nutrients, and their quantities in the media depend on the content of these nutrients in the microalgal biomass. Nitrogen and phosphorus usually account for 10-20% w/w of microalgal biomass. Additional nutrients present in the medium are considered as macronutrients (Na, Mg, Ca, and K) and micronutrients (Mo, Mn, B, Co, Fe, and Zn) *(*Khan et al., 2018, Microb Cell Fact, 17, 36, https://doi.org/10.1186/s12934-018-0879-x*;* Grobbelaar 2004, Algal nutrition. In: Richmond A, editor. Handbook of microalgal culture: biotechnology and applied phycology. Oxford: Blackwell; 2004. p. 97-115. doi.org/10.1002/9780470995280.ch6). For heterotrophic cultivation, an organic carbon source is required.

According to another particular embodiment, the microalgae are incubated under heterotrophic fermentation conditions in a closed reactor. Typically, heterotrophic fermentation conditions which can be used is an incubation time from about 3 days to about 2 weeks, at a temperature from about 20°C to about 35°C, e.g., 26°C.

According to another particular embodiment, the fructose concentration in the culture medium is monitored continuously or in a regular manner at a frequency from about 1 to about 2 times per day. Typically, the fructose concentration in the culture medium can be measured by high pressure liquid chromatography (HPLC). For example, glucose and fructose concentrations are measured by using an Agilent 1100 HPLC equipped with an Aminex HPX-87H ion exclusion column (300 mm x 7.8 mm) and a RID detector. Sulfuric acid 5 mM, used as the solvent, is flowed at 0.6 mL/min through the column heated at 40°C. For the sample analysis, 1 mL samples are filtered using Chromafil Xtra PA filters 0.45 µm and placed in a vial. Standards can be prepared to quantify the amount of sucrose, glucose and fructose (0.5, 1, 2, 4, 8, 10 g·L-1).

According to another particular embodiment, the fermentation process is stopped when the fructose content reaches a target level of not lower than 1 g/L. The microalgal biomass is harvested and processed.

According to another particular embodiment, the harvesting of the microalgae material from the culture medium is carried out by centrifugation. However, other technologies can be used, as described in Barros et al., 2015, Renewable and Sustainable Energy Reviews, 41, 1489-1500. doi: 10.1016/j.rser. 2014.09.037*.*

According to another particular embodiment, the washing of the harvested microalgae material is carried out by washing the microalgae material, such as with water or with an isotonic salt solution, optionally comprising fructose, for removing the rest of the culture medium while preserving as much as possible the fructose content of the microalgal biomass to ensure a minimum content of fructose in the final product. Typically, the washing of the algal biomass is carried out with an isotonic solution such as 0.9% NaCl at low temperature (typically below 20°C such as at 4°C) and separation from the washing solution is performed gently such as through a gentle centrifugation e.g., from 1'000 rcf to 5'000 rcf, e.g., 5'000 rcf. Washing can be done with different isotonic solutions or with water, based on the final use of the biomass or the characteristics of the culture medium. In some production processes, washing could be avoided.

According to a particular aspect of the invention, the microalgae is any microalgae suitable for mammal an in particular human consumption.

According to a particular aspect of the invention, the microalgae is from the phylum *Chlorophyta.*

According to a particular aspect of the invention, the microalgae is *Chlorella.*

According to a particular aspect of the invention, the microalgae is *Chlorella vulgaris.* According to a particular aspect of the invention, the method of the invention further comprises a drying step of the obtained microalgae material.

Another particular aspect of the invention, is to provide a method or a process for manufacturing a microalgae material, wherein the said microalgal material contains at least 10% of proteins.

According to another particular aspect of the invention, is provided a method according to the invention wherein the obtained high-protein microalgae material contains at least 0.2% fructose resulting from the fermentation process or from the washing step.

According to another particular aspect of the invention, is provided microalgae product wherein the chlorophyll content is below 2% w/w.

According to another particular aspect of the invention, is provided a *Chlorella vulgaris* microalgae product of the invention having minimum protein content of at least 20%, 25%, 30%, 35%, 40%, 45%, or 50% w/w.

According to another particular aspect of the invention, is provided a food or a feed product comprising a microalgae material according to the invention. The microalgal biomass can be then used as ingredient in different food products such as pasta, backed goods, beverages, meat substitutes, dairy substitutes, etc., where the microalgal biomass is incorporated as source of protein, natural colorant or to increase the nutritional value of the product. In addition, the microalgae product can be used as food supplements in form of capsules, pills, etc.

Examples illustrating the invention will be described hereinafter in a more detailed manner and by reference to the embodiments represented in the Figures.

### EXAMPLES

### Example 1: Medium preparation using molasses and sugar syrup as carbon source for a method of the invention

Molasses and sugar syrup can be used as carbon sources after a hydrolysis step to obtain a mixture of different monosaccharides, including fructose and glucose. Typically, the hydrolysis is performed by dissolving molasses and sugar syrup in water and adding different concentrations of sulfuric acid (H₂SO₄). Subsequently, the solution is autoclaved at 121 °C for 20 min. **Table 1** below shows different hydrolysis conditions applied to molasses and sugar syrup to convert sucrose into an equimolar mixture of glucose and fructose. Untreated molasses and sugar syrup samples contained more than 50% of sucrose. After hydrolysis, the fraction of sucrose hydrolyzed into glucose and fructose was measured to determine the conversion (%), and the ratio glucose to fructose was measured to determine the degradation of sugars during hydrolysis. Hydrolysis conditions are chosen based on the higher conversion (>95%) and a ratio glucose/fructose close to 1.

**Table 1**

| Sample | **Conversion [%]** | **Ratio glucose/fructose [-]** |
|---|---|---|
| Molasses 100 g/L with H₂O | **5,20** | **-** |
| Molasses 100 g/L with H₂SO₄ 50 mM | **93,29** | **0,94** |
| Molasses 100 g/L with H₂SO₄ 100 mM | **94,29** | **0,98** |
| Molasses 200 g/L with H₂O | **9,73** | **-** |
| Molasses 200 g/L with H₂SO₄ 50 mM | **54,96** | **0,90** |
| Molasses 200 g/L with H₂SO₄ 100 mM | **94,19** | **0,94** |
| Molasses 200 g/L with H₂SO₄ 250 mM | **94,01** | **1,03** |
| Molasses 300 g/L with H₂O | **3,85** | **-** |
| Molasses 300 g/L with H₂SO₄ 50 mM | **30,33** | **0,93** |
| Molasses 300 g/L with H₂SO₄ 100 mM | **74,45** | **0,90** |
| Molasses 300 g/L with H₂SO₄ 250 mM | **93,04** | **0,99** |
| Sugar syrup 300 g/L with H₂O | **3,69** | **-** |
| Sugar syrup 300 g/L with H₂SO₄ 50 mM | **98,67** | **0,94** |
| Sugar syrup 400 g/L with H₂O | **1,36** | **-** |
| Sugar syrup 400 g/L with H₂SO₄ 50 mM | **97,34** | **0,94** |
| Sugar syrup 400 g/L with H₂SO₄ 100 mM | **98,00** | **0,96** |
| Sugar syrup 500 g/L with H₂O | **-0,55** | **-** |
| Sugar syrup 500 g/L with H₂SO₄ 50 mM | **78,43** | **0,96** |
| Sugar syrup 500 g/L with H₂SO₄ 100 mM | **98,33** | **0,96** |
| Sugar syrup 600 g/L with H₂O | **0,92** | **-** |
| Sugar syrup 600 g/L with H₂SO₄ 50 mM | **50,56** | **1,06** |
| Sugar syrup 600 g/L with H₂SO₄ 100 mM | **98,22** | **0,96** |

In the process here described, hydrolysis of molasses was carried out with 300 g/L of molasses in 250 mM of H₂SO₄, and the hydrolysis of sugar syrup was carried out with 600 g/L sugar syrup in 100 mM of H₂SO₄. The preferred conditions are chosen in order to achieve conversion rates of at least 95 %, which indicates that the major fraction of sucrose is hydrolyzed, and a ratio glucose/fructose close to 1, which indicates that glucose and/or fructose were not decomposed.

### Example 2: C. vulgaris and C. protothecoides cultivation using different carbon sources

### in batch cultivation

The method according to the invention was used for production of C. *vulgaris* or C. *protothecoides,* wherein said method comprises the following steps:

### Providing a microalgal inoculum to a microalgae culture medium in batch cultivation

The same inoculum was prepared for both microalgae strains, containing the same nutrients than in the culture medium used for cultivation. The only difference between the nutrient composition in the inoculum and the culture medium is the carbon source, which was provided with pure glucose in the inoculum.

The culture medium was prepared with the following nutrients:
1.637 g/L NaH₂PO₄, 1.980 g/L K₂HPO₄, 1.092 g/L citric acid, 1.232 g/L MgSO₄x7H₂O, 0.023 g/L CaCl₂x2H₂O, 1 g/L (NH₄)₂SO₄, 1.83 x 10⁻³ g/L H₃BO₃, 3.52 x 10⁻³ g/L ZnSO₄x7H₂O, 2.46 x 10⁻³ g/L MnSO₄xH₂O, 9.60 x 10⁻⁵ g/L NaMoO₄x2H₂O, 8 x 10⁻⁵ g/L Ni(NO₃)₂6H₂O, 9.88 x 10⁻⁵ g/L CuSO₄x5H₂O, 1.50 x 10⁻³ g/L FeSO₄x7H₂O, 9.98 x 10⁻⁴ g/L ThiaminexHCl (Vit B1), 5.34 x 10⁻⁵ g/L D-Pantothenic acid hemicalcium salt (Vitamin B5 calcium salt), 1.60 x 10⁻⁶ g/L Biotin (Vit H), 1.12 x 10⁻⁷ g/L Cyanocobalamin (Vit B12), 5.01 x 10⁻⁶ g/L Riboflavin (Vit B2), 2.61 x 10⁻⁶ g/L PyridoxinexHCl (Vit B6), Carbon source: in some of the experiments, pure glucose and fructose were supplied in order to have a total carbon targeted at 16 g carbon/L. When hydrolyzed molasses and sugar syrup were used as carbon source, the calculation was made based on the glucose and fructose concentration in both substrates; the total carbon targeted in the media was also 16 g carbon/L.

### Supplying the microalgae culture medium with at least 1 g/L of fructose

Hydrolyzed molasses/sugar syrup, containing glucose and fructose, was added in order to have a total carbon targeted at 16 g carbon/L. **Figures 2** and **3** show the growth of two different microalgae strains, *C*. *vulgaris* and *C. protothecoides* respectively. The figures show the growth of both strains when the culture medium is prepared with hydrolyzed molasses/sugar syrup as carbon source according to the present invention. As matter of comparison, cultivation of both strains with pure glucose and pure fructose is also shown in the figures. When comparing the growth in fructose, **Figure 3C** reveals that the C. *vulgaris* strain used cannot metabolize fructose.

### Incubating the microalgae under heterotrophic fermentation conditions in the culture medium

The microalgae mixtures were incubated in batch reactors from 50 mL to up to 2 L. An inoculum is provided of about 1% v/v in order to have an approximate cell concentration of around 5E06 Cells/mL, the pH of the medium was initially adjusted to 6, temperature controlled at 26°C and with air provided by a vented cap.

### Stopping the cultivation when the fructose content of the microalgae culture medium was at least 1 g/L

The content of fructose and other carbon sources was monitored by HPLC. The cultivation was stopped and the biomass centrifuged when the preferred carbon source, i.e., glucose, was depleted in the medium or no significant growth was observed. In the case of C. *vulgaris,* when the cultivation was stopped, the concentration of fructose was around 20 g/L. For C. *protothecoides,* cultivation was stopped when the concentration of fructose was around 8 g/L.

### Harvesting the microalgae material from the reactor

The obtained microalgae were harvested from the reactor by centrifugation.

**Figure 2** shows the growth of C. *vulgaris* as measured spectrophotometrically and the variation in the culture medium of the contents in various types of sugar as measured by HPLC when C. *vulgaris* is cultured by a method according to the invention and using various carbon sources such as hydrolyzed molasses, sugar syrup, glucose or fructose.

The results show that C. *vulgaris* grows in hydrolyzed sugar syrup, reaching higher productivity than in the medium prepared only with glucose as consumable source of carbon, but is unable to grow in the medium prepared with fructose as the only source of carbon.

The same method and conditions were applied to the growth of a *C. protothecoides* strain and **Figure 3** shows that the consumption of the different sugars is strain dependent. C. *protothecoides* is able to grow on hydrolyzed molasses and also to metabolize fructose during growth, as opposed to C. *vulgaris.*

Therefore, the results show that *C. protothecoides* grow in hydrolyzed molasses and hydrolyzed sugar syrup, reaching similar productivity than in a medium prepared only with glucose as the only source of carbon.

### Example 3: C. protothecoides cultivation using hydrolyzed sugar syrup as carbon source in fed-batch condition

The same method as described in Example 2 were applied to the growth of a C. *protothecoides* strain in a fed-batch cultivation in order to support that the method of the invention can be applied to cultivation in batch, semi-continuous or continuous mode. Preparation of the inoculum, inoculation and culture medium were the same than described in Example 2. In the fed-batch cultivation, microalgae were inoculated in a reactor containing initially 1.5 L of medium, at a pH initially adjusted to 6, a temperature of 26°C, and an air flow rate through the reactor of 1 vvm (volume of air going through aeration per volume of culture liquid per minute: i.e., in 1 minute time there is 0.5 liter of air passing through 1 liter of medium in 1 minute of time).

As carbon source, the fed-batch cultivation was performed using hydrolyzed sugar syrup. Microalgae growth was monitored through the absorbance at 800 nm and through the measurement of dry cell weight. The variation of the sugar content in the culture medium is measured by HPLC. When the concentration of nutrients was low so that it reduced the microalgae growth rate, an exponential feeding of nutrients was adopted to prolong the exponential phase and increase the biomass productivity.

**Figure 4** shows that high density culture of *C*. *protothecoides* can be reached using hydrolyzed sugar syrup fed in an exponential manner.

Therefore, the results show that the method according to the invention can be applied to different cultivation regimes like fed-batch, where the carbon source can be added in consecutive steps during cultivation.

### Stopping the fermentation process when the fructose content of the microalgae culture medium is about 1 g/L w/w

The content of fructose in the reactor was monitored by HPLC. Based on the microalgae growth, the fermentation was stopped by centrifuging the culture medium, ensuring that the fructose content was higher than 1 g/L.

### Harvesting the microalgae material from the reactor

The obtained microalgae were harvested from the reactor by separation of the microalgal biomass from culture broth by centrifugation.

### Optionally washing the harvested microalgae material to obtain a microalgae material with a fructose content that is not less than about 0.2% w/w

In function of the final use of the microalgae biomass (food, feed or extraction of high-value compounds like pigments) and the regulations about the quality of the product, the washing step can be adapted accordingly. The harvested microalgal biomass was then washed with isotonic salt solution for removing the rest of the culture medium while preserving as much as possible the fructose content of the microalgal biomass to ensure a minimum content of fructose in the final product. Comparative conditions are presented under Example 4.

After the biomass is washed, various drying techniques can be applied to obtain the final product in the form of microalgal powder, as described by De Farias Neves et al., 2020, Drying and Quality of Microalgal Powders for Human Alimentation. Microalgae-From Physiology to Application. doi:10.5772/intechopen.89324*.* For example, spray drying can be used to obtain a fine powder such as described in Hosseinizand et al., 2018, Drying Technology, An International Journal, 36(9), 1049-1060*.* Drum drying can be also used to obtain flakes that can be milled for producing a fine microalgal powder. For some applications, the wet biomass could be directly used without drying.

### Example 4: C. vulgaris cultivation using hydrolyzed sugar syrup as carbon source in batch condition

The method according to the invention was used for production of C. *vulgaris,* wherein said method comprises the following steps:

### Providing a microalgal inoculum to a microalgae culture medium in a reactor

The inoculum was prepared in a medium containing the same nutrients than in the culture medium used for cultivation. The only difference between the nutrient composition in the inoculum and the culture medium is the carbon source, which was provided with pure glucose in the inoculum. The culture medium was prepared with the following nutrients:
KNO₃: 12.0 g/L, MgSO₄x7H₂O: 3.30 g/L, KH₂PO₄: 2.00 g/L, CaCl₂x2H₂O: 0.175 g/L, citric acid: 1.00 g/L, glucose: 40 g/L, EDTAxNa₂x2H₂O: 84.5 mg/L, FeSO₄x7H₂O: 0.120 g/L, H₃BO₃: 18.5 mg/L, CoSO₄x7H₂O: 8.40 mg/L, CuSO₄x5H₂O: 7.40 mg/L, (NH₄)₆Mo₇O₂₄x4H₂O: 6.00 mg/L, MnSO₄x4H₂O: 7.00 mg/L, ZnSO₄x7H₂O: 8.60 mg/L. Carbon source: hydrolyzed sugar syrup for a total carbon targeted at 16 g carbon/L

### Supplying the microalgae culture medium with at least 1 g/L of fructose

The microalgae were inoculated in a reactor containing 2 L of medium, at a pH initially adjusted to 7, a temperature of 26°C, and an air flow rate through the reactor of 0.5 vvm (volume of air going through aeration per volume of culture liquid per minute: i.e., in 1 minute time there is 0.5 liter of air passing through 1 liter of medium in 1 minute of time).

As carbon source, the cultivation was performed using hydrolyzed sugar syrup. Microalgae growth was monitored through the absorbance at 800 nm and through the measurement of dry cell weight. The variation of the sugar content in the culture medium is measured by HPLC.

### Stopping the cultivation process when the fructose content of the microalgae culture medium is about 1 g/L w/w

The content of fructose in the reactor was monitored by HPLC. Based on the microalgae growth, the fermentation reaction was stopped by centrifuging the culture medium, ensuring that the fructose content was higher than 1 g/L.

The same method in Example 3 were applied to the harvest and washing of the microalgae biomass. **Figure 5** shows that C. *vulgaris* can be successfully grown using hydrolyzed sugar syrup and that the method according to the invention can be applied to different cultivation regimes like batch or fed-batch, where the carbon source can be added in consecutive steps during cultivation. The microalgae do not consume the fructose, thus remaining in the medium at the end of the fermentation.

### Example 5: evaluation of different washing conditions for microalgae after fermentation in a method according to the invention

For the quantification of fructose, an exact amount of microalgal biomass is suspended in an exact amount of water. The suspension is then sonicated to ensure a proper dispersion of the particles. The suspension is then centrifuged, and the fructose in the supernatant is analyzed using an HPLC and a calibration curve. The result is expressed as the quantity of fructose in the microalgal sample (expressed as dry biomass). This methodology is applied to quantify the fructose in different steps of the process, e.g., microalgal biomass recovered from the reactor (before washing), the microalgal biomass recovered after different washing steps or the microalgal biomass after drying (final product).

It is important to note, according to the invention, that washing of the harvested microalgae material has to be carried out carefully in order to obtain an isolated microalgae material with a fructose content that is not less than about 0.2% w/w.

In this particular case, the washing of the algal biomass is performed in an isotonic solution such as 0.9% NaCl at low temperature (typically from 4°C to about 16°C such as at 4°C) and that separation from the washing solution is performed gently such as through a gentle centrifugation e.g. 5'000 rcf.

Further, the number of washing steps affects the final content in fructose in the product. For example, in the particular case, the number of washing steps following the procedure described in Example 4 should not exceed 2 as can be seen in **Figure 6****,** where the influence of the number of washing steps on the content of fructose in the final product was quantified for washing steps caried out with a solution of NaCl 0.9 % at 4 °C. Separation was performed via centrifugation at 5'000 rcf. Different numbers of washing steps were evaluated and the content of fructose in the final product was quantified. When C. *vulgaris* was washed more than twice, the content of fructose in the final product was below the desired value. Alternatively, the washing steps could be conducted in presence of fructose in the washing solution to ensure that a minimum of 0.2 % w/w of fructose remains in the product.

### Example 6: Characterization of the obtained microalgae product

Different analyses are performed to determine the quality of the biomass produced according to a method described in the invention. These analyses include the determination of the content of fructose, pigments (chlorophyll and carotenoids), and protein.

The quantification of fructose in the final microalgal product was done following the same methodology than in **Example 5,** to ensure a fructose content of at least 0.2 % w/w.

For the quantification of proteins, the nitrogen content in the biomass is analyzed using a total nitrogen analyzer, and the content of protein is then calculated by multiplying the nitrogen content by a conversion factor (N * 6.25). The final product was analyzed to ensure a protein content higher than 10 % w/w.

For the quantification of pigments, the method described by *Pruvost et al., 2011, supra* is used. Basically, the method requires the extraction of the pigments using an organic solvent, and the quantification of the pigments in the extract using a spectrophotometer.

Table 2 below compares the cultivation of C. *vulgaris* under four different cultivation conditions: three cultivations under heterotrophic conditions with different carbon sources and one cultivation under autotrophic conditions. The microalgal biomass from heterotrophic conditions resulted in a product with a lower chlorophyll content than under autotrophic conditions. The heterotrophic conditions also resulted in a decreased carotenoid content, however, their proportion in the total pigments (chlorophyll + carotenoids) increased. When focusing on heterotrophic cultivation, the results in Table 2 also show that the presence of fructose (either supplied directly or through the use of hydrolyzed sugar syrup) contributes to reduce the chlorophyll content and the proportion of carotenoids (ratio between the concentration of carotenoids and the total concentration of pigments, being carotenoids and chlorophylls) in the product.

**Table 2**

| | | | | |
|---|---|---|---|---|
| **Conditions** | **Organic carbon source** | **Total chlorophyll (Chl) [%]** | **Total carotenoids (Car) [%]** | **Car/(Car + Chl)) [-⁻]** |
| Heterotrophic | Glucose | 1,95 | 0,421 | 0,178 |
| | Glucose + Fructose | 1,85 | 0,427 | 0,187 |
| | Hydrolyzed sugar syrup | 1,49 | 0,376 | 0,202 |
| Autotrophic | - | 6,04 | 1,22 | 0,168 |

## Claims

1. A method for production of microalgae under heterotrophic fermentation conditions, wherein said method comprises the step of stopping the fermentation process when the fructose content in the microalgae culture medium reaches a target level, said target level being at least 1g/L.

2. A method for production of microalgae according to claim 1, wherein said method comprises the following steps:
- Providing a microalgal inoculum to a microalgae culture medium;
- Supplying the microalgae culture medium with at least 1 g/L of fructose;
- Incubating the microalgae under heterotrophic fermentation conditions in the culture medium;
- Stopping the fermentation process when the fructose concentration of the microalgae culture medium is at least 1 g/L;
- Harvesting the microalgae material from the culture medium;
- Optionally washing the harvested microalgae material to obtain a concentrated microalgae material with a fructose content that is not less than about 0.2% w/w.

3. A method according to claim 2 wherein an inoculum is provided under step a) at a concentration of about 1 v/v.

4. A method according to anyone of the preceding claims wherein the fructose is provided under heterotrophic fermentation conditions in the form of a carbon source which contains fructose and/or a carbon source that generates fructose when hydrolyzed.

5. A method according to anyone of claims 1 to 3 wherein the fructose provided under heterotrophic fermentation conditions though the direct addition of fructose in the culture medium.

6. A method according to anyone of the preceding claims wherein the heterotrophic fermentation conditions comprise an incubation time from about 3 days to about 2 weeks, at a temperature from about 20°C to about 35°C, e.g., 26°C.

7. A method according to anyone of the preceding claims wherein the fructose concentration in the culture medium is monitored continuously or in a regular manner at a frequency from about 1 to about 2 times a day.

8. A method according to anyone of the preceding claims wherein the fermentation process is stopped when the fructose content reaches is not lower than 1g/L.

9. A method according to anyone of the preceding claims wherein harvesting of the microalgae material from the culture medium is carried out by centrifugation.

10. A method according to anyone of the preceding claims wherein the washing of the harvested microalgae material is carried out by washing the microalgae material with isotonic salt solution for removing the rest of the culture medium while preserving as much as possible the fructose content of the microalgal biomass to ensure a minimum content of fructose in the final product.

11. A method according to anyone of the preceding claims wherein the microalgae is any microalgae suitable for mammal and in particular human consumption, e.g., from the phylum *Chlorophyta such as Chlorella,* in particular *Chlorella vulgaris.*

12. A method according to anyone of the preceding claims, wherein said method further comprises a drying step of the obtained microalgae material.

13. A microalgae material obtainable from a method according to any one of claims 1 to 12.

14. A microalgae material having at least one of a yellow, orange, white or red pigment and wherein the chlorophyll content is below 2% w/w.

15. A food or a feed product comprising a microalgae material according to claims 13 or 14.
